# EUROPÄISCHE PATENTSCHRIFT

(11) **EP 3 603 547 B1**
(45) Veröffentlichungstag und Bekanntmachung des Hinweises auf die Patenterteilung: **21.07.2021**
(21) Anmeldenummer: 19194912.2
(22) Anmeldetag: 20.05.2015
(51) Int. Cl.: A61B 17/3205, A61B 17/32, A61B 18/14, A61B 17/22, A61B 18/00, A61B 17/00

(54) **PAPILLOTOM FÜR DIE PERKUTANE ENDOSKOPISCHE GASTROSTOMIE**
PAPILLOTOME FOR PERCUTANEOUS ENDOSCOPIC GASTROSTOMY
PAPILLOTOME POUR LA GASTROSTOMIE ENDOSCOPIQUE PERCUTANÉE

(30) Priorität: 10.06.2014 DE 102014211048; 09.09.2014 DE 102014112985
(43) Veröffentlichungstag der Anmeldung: 05.02.2020
(62) Teilanmeldung aus: 15741501.9
(73) Patentinhaber: FUJIFILM MEDWORK GMBH, 91315 Höchstadt/Aisch (DE)
(72) Erfinder: Dormann, Prof. Dr. Arno, 51069 Köln (DE); Brehm, Andreas, 91325 Adelsdorf (DE); Klyeisen, Jörg, 91336 Heroldsbach (DE); Stirnweiß, Matthias, 91475 Lonnerstadt (DE)
(74) Vertreter: Gosdin, Carstensen & Partner Patentanwälte Partnerschaftgesellschaft mbB

(56) Entgegenhaltungen:
- DE-A1-102011 085 721
- US-A- 5 163 938
- US-A- 5 396 902
- US-A- 6 017 340
- US-A1- 2008 091 196

## Beschreibung

Die Erfindung betrifft ein Papillotom für die perkutane endoskopische Gastrostomie (PEG), das aufweist a) einen Handgriff, b) einen länglichen, biegbaren Katheter, der am Handgriff befestigt ist, der einen freien Endbereich mit einem freien Ende und der mindestens ein erstes Lumen aufweist, c) eine vordere Öffnung, die sich im freien Endbereich befindet und das erste Lumen mit einer Außenseite des Katheters verbindet, d) eine hintere Öffnung, die sich im freien Endbereich befindet, die weiter entfernt vom freien Ende ist als die vordere Öffnung und die das erste Lumen mit der Außenseite verbindet, wobei die vordere Öffnung und die hintere Öffnung in derselben Orientierung zum Katheter angeordnet sind, und e) einen Schneidedraht, der sich axial verschiebbar im ersten Lumen befindet, der durch die vordere Öffnung und die hinteren Öffnung verläuft, der sich zwischen der vorderen Öffnung und der hinteren Öffnung auf der Außenseite befindet und der im freien Endbereich und im Handgriff festgelegt ist, wobei bei betätigtem Handgriff der Schneidedraht gespannt ist, der freie Endbereich bogenförmig verformt ist und der Schneidedraht zwischen der vorderen Öffnung und der hinteren Öffnung eine quer verlaufende Schnittkante bildet, wobei die vordere Öffnung in einem Abstand von mindestens 3 mm vom freien Ende angeordnet ist und zwischen dem freien Ende und der vorderen Öffnung ein Überstand ausgebildet ist.

Aus DE 36 43 362 A1, DE 26 57 256 A1 und DE 94 09 072 U1 sind Papillotome bekannt. Sie werden endoskopisch in den Körper eines Patienten eingeführt. Insbesondere sind sie zur Entfernung von Gallensteinen vorgesehen. Sie werden auch als Schlingen-Papillotome bezeichnet.

Bei den vorbekannten Papillotomen befindet sich eine vordere Öffnung am freien Ende des Katheters. Der Schneidedraht tritt an diesem freien Ende aus und befindet sich bis zur zweiten Öffnung an der Außenseite des Katheters. Durch Betätigen des Handgriffs wird der Schneidedraht gegenüber dem Katheter verschoben und der Katheter zwischen der vorderen und einer zweiten, hinteren Öffnung gebeugt bzw. U-förmig verformt. Der Schneidedraht verbindet die vordere Öffnung mit der zweiten Öffnung direkt. Zwischen Schneidedraht und dem gebeugten freien Endbereich des Katheters wird eine Schlinge ausgebildet. Der Schneidedraht ist mit einer Hochfrequenz (HF)-Spannungsquelle verbunden. In bekannter Weise und nach dem Stand der Technik kann mit dem Schneidedraht geschnitten werden. Der Schnittweg wird durch die Bewegung des Katheters bestimmt.

Schlingen-Papillotome dieser Art haben sich speziell für die endoskopische Anwendung, insbesondere zur Entfernung von Gallensteinen, bewährt. Dabei wird in der Regel der papilla vateri bzw. der musculus sphincter oddi durchtrennt. Gewebe, das über ein gewisses Niveau herausschaut, kann durchtrennt werden.

Ein Papillotom der im Oberbegriff des Anspruchs 1 genannten Gattung, das im Wesentlichen mit den vorgenannten Merkmalen ausgeführt und für diese Anwendung vorgesehen ist, geht aus der US 6 017 340 A hervor. Ein derartiges Papillotom soll demzufolge in der endoskopischen Sphinkterotomie in Verbindung mit einem Endoskop verwendet werden, um einen chirurgischen Schnitt innerhalb eines Patienten zu ermöglichen. Vorzugsweise wird ein derartiges Papillotom verwendet werden, um das Duodenum an der papilla vateri teilweise aufzuschneiden, so dass Gallensteine, die eine Obstruktion bilden, entfernt werden können. Bei der endoskopischen Sphinkterotomie können größere Komplikationen wie Blutungen, Pankreatitisperforation und Cholangitis auftreten. Ein Schneidedraht des Papillotoms ist mit einem axialen Abstand zum distalen Ende des Katheters an diesem fixiert, wobei bei einem Ausführungsbeispiel der Katheter mit einer modifizierten distalen Spitze versehen ist, um mittels dieses Profils die Kanülierung des Schließmuskels der Papille zu erleichtern.

Aus der DE 10 2011 085 721 A1 ist eine asymmetrisch öffnende und schließende HF-chirurgische Resektionsschlinge bekannt, die einen isolierten, gekrümmt verlaufenden Schlingenabschnitt und einen eine Schneidkante bildenden nichtisolierten Schlingenabschnitt aufweist. Dabei erstreckt sich die Resektionsschlinge über ein distales Ende eines Katheters hinaus. Ein Übergang vom isolierten Schlingenabschnitt zum nichtisolierten Schlingenabschnitt soll dabei die Form eines Sporns aufweisen, der für einen gewissen Abstand des nichtisolierten Schlingenabschnitts zum Gewebe und somit unbeabsichtigtes Schneiden sorgen soll.

Aus der US 2008/0091196 A1 geht ein sogenanntes Nadelmesser hervor, das in Verbindung mit einem Endoskop verwendet wird, um einen chirurgischen Schnitt innerhalb eines Patienten durchzuführen. Beispielsweise ist eine Verwendung für eine Sphinkterotomie vorgesehen, bei der eine Inzision eines Schließmuskels erfolgt. Insbesondere handelt es sich dabei um die Entfernung von Gallensteinen aus dem Hauptgallengang. Das distal am Katheter angeordnete Nadelmesser kann außer für die Schneidfunktion auch für eine Flüssigkeitskommunikation (z.B. Spülflüssigkeit, Radiokontrastmittel) sowie das Absaugen von Gewebe verwendet werden und dafür hohl ausgebildet sein. Mittels eines in einem Lumen verlaufenden und mit dem distalen Ende des Katheters verbundenen Zugdrahtes wird das distale Ende des Katheters und somit das Nadelmesser gesteuert. Alternativ kann der Zugdraht auch elektrisch leitend sein und die Funktion eines Schneiddrahtsphinkterotoms übernehmen.

Die US 5 396 902 A betrifft eine Drahtführungs- oder Sondenanordnung zum Einführen eines medizinischen Katheters oder elektrischer Zuleitungen zu einem gewünschten Ort innerhalb des Körpers eines Patienten. Dabei geht es darum, eine einfache und einfach handhabbare Sonden- und Manipulationsgriff-Anordnung zu schaffen, um dem distalen Ende einer Zuleitung oder eines Katheters während des Vorschiebens durch ein Blutgefäß eine dynamische Krümmung zu verleihen. Ein in einem Lumen des Katheters verlaufender Zugdraht soll ähnlich wie bei einem Papillotom mechanisch mit der distalen Spitze zusammenwirken. Dabei soll dieser bis zum distalen Ende des Katheters verlaufen und dort fixiert sein. Dabei tritt der Zugdraht über zwei Öffnungen aus dem Lumen aus bzw. wieder in dieses ein, verläuft zwischen diesen Öffnungen außen entlang des Katheters.

Bei einem Buried Bumper-Syndrom geht es um eine eingewachsene PEG-Halteplatte einer PEG-Sonde. Eine PEG-Sonde wird dem Patienten durch die Bauchdecke und die Magenwand (perkutan) in den Magen eingesetzt. Über diese Sonde wird der Patient mit Nahrung und Flüssigkeit versorgt. Die PEG-Sonde weist einen PEG-Katheter auf. Dieser wird außerhalb des Körpers mit einem Anschlusssystem der PEG-Sonde verbunden, das dann über den PEG-Katheter mit dem Mageninneren verbunden ist. Der PEG-Katheter verläuft durch die Bauchdecke und die Magenwand. Bei unzureichender oder falscher Pflege kann es dazu kommen, dass die PEG-Halteplatte von der Magenschleimhaut der Magenwand (Magenmukosa) umschlossen und überwachsen wird. Dies wird als Buried Bumper Syndrom bezeichnet.

Nach dem Stand der Technik werden eingewachsene PEG-Halteplatten per Laparotomie, mittels des Pull-Verfahrens oder des Push-Verfahrens entfernt.

Unter einer Laparotomie versteht man die chirurgische Öffnung der Bauchdecke. Im Zuge dieser Operation wird auch die Magenwand geöffnet und die eingewachsene PEG-Halteplatte von außen freigeschnitten. Dies ist eine aufwändige, stark invasive und daher risikoreiche Operation, die nur noch in seltenen Fällen angewandt wird.

Das Pull-Verfahren basiert darauf, die eingewachsene PEG-Halteplatte nach vorherigem Einschneiden des umgebenden Gewebes durch Zug von außen aus dem Gewebe zu befreien. Durch den liegenden PEG-Katheter wird eine Biopsie Zange zur Versteifung desselben eingeführt und als Widerstand geöffnet. Durch Zug auf die Biopsiezange wölbt sich die Bauchdecke an der Stelle der eingewachsenen PEG-Halteplatte. Mit einem Skalpell wird die Bauchdecke und ein Teil der Magenwand in der direkten Umgebung des PEG-Katheters bis auf die PEG-Halteplatte durchtrennt. Anschließend wird die PEG-Halteplatte durch Zug von außen, mittels der Biopsiezange nach außen freigezogen. Dieses Verfahren ist weniger invasiv als eine Laparotomie, jedoch wird auch hierbei noch ein Teil der Bauchdecke und der Magenwand durchtrennt, dies ist mit den damit verbundenen Risiken behaftet.

Beim Push-Verfahren wird unter endoskopischer Kontrolle die PEG-Halteplatte von innen, also aus dem Mageninneren heraus, frei geschnitten. Dazu wird endoskopisch oder perkutan ein Schneidewerkzeug in das Mageninnere eingeführt und die PEG-Halteplatte freigelegt. Zumeist werden dafür Nadel-Papillotome verwendet, diese besitzen an ihrer Spitze ein nadelartiges Schneidwerkzeug, mit dem das Gewebe durchtrennt werden kann. Sie werden meist endoskopisch eingeführt. Es können auch Schlingen-Papillotome verwendet werden, diese werden perkutan über den PEG-Katheter eingeführt. Dazu werden sie im entspannten Zustand, nicht gebeugt, durch den PEG-Katheter vorgeschoben. Sobald der Papillotom-Katheter weit genug ins Mageninnere (Magenlumen) vorgeschoben ist, wird der Handgriff betätigt und somit die Papillotomschlinge gebildet. Durch Zurückziehen des Katheters wird der Schneiddraht auf das die PEG-Halteplatte umgebende Gewebe gezogen, dieses kann nun eingeschnitten werden, bis der Schneiddraht auf die PEG-Halteplatte stößt. Meist erfolgen mehrere dieser radiären Schnitte, bis die PEG-Halteplatte freigelegt ist. Anschließend wird die PEG-Halteplatte mit einem durch den PEG-Katheter vorgeschobenen handelsüblichen Bougie, oder auch Dilatator, in das Mageninnere hinein gedrückt. Die befreite PEG-Halteplatte kann dann endoskopisch entfernt werden.

Bei Bougies handelt es sich um Körperöffnungen oder Gefäß erweiternde medizinische Geräte zumeist aus Edelstahl oder anderen harten Materialien mit einer konischen Form.

Das Push-Verfahren ist das am wenigsten invasive Verfahren und bietet die geringste Komplikationsrate von den dargestellten Verfahren. Allerdings treten auch hier Probleme und Komplikationen auf. Nachteile der Push Technik sind ein häufig vorkommendes Abrutschen bzw. Verrutschen der Schlinge des Papillotoms beim Zurückziehen auf die PEG-Halteplatte, dadurch werden stärkere Blutungen, größere Infektionsherde und ein höherer Arbeitsaufwand ausgelöst. Weiterhin kann die PEG-Halteplatte seitlich nicht frei gelegt werden. Die vorhandenen Bougies sind nicht für die Anwendung mit einer und für eine PEG-Sonde ausgelegt.

Hiervon ausgehend ist es Aufgabe der Erfindung, für das Push-Verfahren eine Vorrichtung zur Verfügung zu stellen, mit der der Eingriff zielgerichteter und einfacher durchführbar ist, ein Verrutschens des Papillotoms vermieden wird und die Schnitte gezielter gesetzt werden können.

Diese Aufgabe wird gelöst durch eine Vorrichtung, die zusätzlich zu den oben genannten Merkmalen noch folgende Merkmale enthält: Am freien Ende des Katheters ist eine Schneidspitze ausgebildet, die mit dem Schneidedraht verbunden ist und das vorderste Ende des Papillotoms bildet, wobei der Schneidedraht sich zudem zwischen dem freien Ende und der vorderen Öffnung auf der Außenseite befindet, dort die Schneidspitze und eine längs verlaufende Schnittkante bildet und dort in derselben Orientierung zum Katheter wie die vordere Öffnung und die hintere Öffnung angeordnet ist. Somit ist die vordere Öffnung in einem Abstand von mindestens 3 mm vom freien Ende angeordnet, und zwischen dem freien Ende und der vorderen Öffnung ist ein Überstand ausgebildet. Der Schneidedraht befindet sich zudem zwischen dem freien Ende und der vorderen Öffnung auf der Außenseite und bildet dort die längs verlaufende Schnittkante, die in derselben Orientierung zum Katheter wie die vordere Öffnung und die hintere Öffnung angeordnet ist. Die längs verlaufende Schnittkante ist durch den Schneidedraht selbst ausgebildet.

Aufgrund des Überstandes beginnt der Schneidedraht nicht unmittelbar am freien Ende des freien Endbereichs des Katheters, sondern im Abstand vom freien Ende. Dadurch kann der Überstand als Anschlag dienen. Nach Einführen des freien Endbereichs in das Mageninnere ist der freie Endbereich noch im Wesentlichen geradlinig. Wird nun der Handgriff betätigt und dadurch der freie Endbereich gebeugt, schlägt der Überstand an einen seitlichen Rand einer Überwachsung der PEG-Halteplatte an. Dadurch kommt es zu einer bestimmten und in gewissem Maße festen Position des Überstandes. Das Papillotom wirkt in seinem freien Endbereich in der Art einer Zange, d.h., zwischen dem Überstand und dem Katheter jenseits der hinteren Öffnung erfolgt ein Festklemmen. Der dort befindliche Teil der PEG-Halteplatte wird übergriffen und eingespannt. Dadurch ist der Schneidedraht auch in Nähe der vorderen Öffnung abgestützt und fixiert. Er ist nicht nur durch den Katheter selbst gehalten. Zieht nun ein Operateur den Katheter nach außen und führt einen Schnitt durch, so schneidet nicht nur bevorzugt derjenige Teil der quer verlaufenden Schnittkante, der sich in Nähe der zweiten Öffnung befindet, wo sich der Zug insbesondere auswirkt, sondern auch derjenige Teil des Schneidedrahtes, der sich in Nähe der vorderen Öffnung befindet, weil dort die beschriebene Abstützung stattfindet. Insbesondere schneidet auch die längs verlaufende Schnittkante, die sich außerhalb des Mantels der scheibenförmigen PEG-Halteplatte befindet. Durch weiteren Zug am Schneidedraht kann die Schlinge verkleinert und somit die Wirkung der längs verlaufenden Schnittkante gesteuert werden. Durch Ziehen am Katheter kann die Wirkung der quer verlaufenden Schnittkante beeinflusst werden.

Der Schneidedraht ragt gegenüber dem freien Ende nach außen vor. Dieser Bereich des Schneidedrahtes bildet eine Schneidespitze. Es ist durchaus möglich und vorgesehen, dass sich die Schneidspitze in einer gewissen Entfernung von z.B. 1-8 mm vom freien Ende des Katheters befindet, also um dieses Maß aus dem ersten Lumen frei hervorragt. Diese erfüllt zudem auch folgenden Zweck: Je nach Stärke der Überwachsung der PEG-Halteplatte kann es vorkommen, dass die innere Öffnung des PEG-Katheters in das Mageninnere überwachsen ist. In diesen Fällen löst und durchschneidet man mit der vorderen Schneidespitze beim Einführen des Katheters die Überwachsung. So kann der Weg in das Mageninnere freigeschnitten werden. Erst danach ist der freie Endbereich des Papillotoms im Mageninneren, Weiterhin ermöglicht die Schneidspitze ein Verhaken in der Magenwand nach bzw. während der Beugung des Papillotoms. Auch hierdurch wird ein Verrutschen des freien Endbereichs verhindert. Ein derartiges Verrutschen kann auch beim Zurückziehen des Katheters auftreten. Es wird ein unkontrolliertes Einschneiden des Schneidedrahtes in die Überwachsung verhindert, zumindest erschwert. Es werden nicht gezielte und unsaubere Schnitte, größere Wundbereiche und somit vermeidbare Blutungen und Infektionsherde vermieden.

Der Überstand kann ausschließlich durch einen Bereich des Katheters gebildet werden.

Die quer verlaufende Schnittkante und die längs verlaufende Schnittkante befinden sich in derselben Orientierung zum Katheter. Hiermit ist folgendes gemeint: Blickt man in Axialrichtung auf den Katheter, so befinden sich die beiden Öffnungen in derselben Winkelposition. Damit befindet sich auch der Schneidedraht beider Schnittkanten in derselben Winkelposition.

Vorzugsweise hat das freie Ende des Katheters eine Abschrägung. Der Winkel der Abschrägung beträgt insbesondere etwa 45°, Abweichungen um ± 20° sind möglich. Durch die Abschrägung wird eine Spitze gebildet. Diese Spitze hat bevorzugt dieselbe Orientierung zum Katheter wie die vordere Öffnung und die hintere Öffnung.

Die Länge des Überstandes entspricht vorzugsweise zumindest der Dicke der verwendeten PEG-Halteplatte. Diese Länge wird der Dicke angepasst. Bei dünnen Halteplatten kann der Überstand kleiner gewählt werden, bei dicken Halteplatten entsprechend größer. Hinzu kommt noch die Dicke der Überwucherung, die aber nicht immer bekannt ist.

Weiterhin kann eine dritte Öffnung im Katheter ausgebildet sein. Sie befindet sich zwischen der vorderen Öffnung und der hinteren Öffnung. Sie ist in unmittelbarer Nähe der vorderen Öffnung, insbesondere 1 bis 8 mm von dieser entfernt. Sie ist ebenso orientiert wie die beiden anderen Öffnungen. Der Draht, der die längs verlaufende Schnittkante bildet, kann durch die dritte Öffnung in den Katheter eingeführt oder aus ihm herausgeführt werden. Die längsverlaufende Schnittkante kann nun so ausgebildet sein, dass sie einen Kreuzungspunkt mit der quer verlaufenden Schnittkante ausbildet. Dadurch wird das Schneiden dort, wo die beiden Schnittkanten zusammenlaufen, verbessert. Die vordere Öffnung wird vorzugsweise nur für den Draht benutzt, der die quer verlaufende Schnittkante ausbildet.

Die quer verlaufende Schnittkante kann auch durch ein Seil gebildet werden. Ein Seil hat den Vorteil, dass es im gebeugten Zustand des freien Bereichs im Wesentlichen geradlinig zwischen den beiden Öffnungen verläuft. Bei einem Schneidedraht, der nur ein Filament hat, wird dies nicht immer vollständig erreicht.

Vorzugsweise ist der Handgriff so ausgebildet, dass der Katheter mit einem stationären Teil des Handgriffs verbunden ist, während ein bewegbarer, insbesondere verschiebbarer Teil des Handgriffs mit dem Schneidedraht verbunden ist. Wird dann der verschiebbare Teil nach außen gezogen, kommt die Beugung zustande.

Der Katheter besitzt vorzugsweise eine Länge zwischen 40 und 150 cm, bevorzugt von 50 bis 120 cm. Handelsübliche Papillotome sind aufgrund ihrer endoskopischen Einführung und der Auslegung für den Gallengang deutlich länger ausgebildet. Eine geringere Länge ermöglicht ein ungehindertes Arbeiten. In bekannter Weise ist der Katheter z.B. aus Polytetrafluorethylen hergestellt. Er hat einen Durchmesser von 2 bis 4 mm. Vorzugsweise hat er zwei Lumina, das zweite Lumen wird für einen Führungsdraht verwendet, ein solcher ist Stand der Technik bei einem Push-Verfahren.

Weitere Vorteile und Merkmale der Erfindung ergeben sich aus den noch nicht angesprochenen Unteransprüchen sowie der nun folgenden Beschreibung mehrerer, nicht einschränkend zu verstehender Ausführungsbeispiele der Erfindung. Diese werden unter Bezugnahme auf die Beschreibung im Folgenden näher erläutert. In der Zeichnung zeigen:
- Fig. 1:: eine Draufsicht auf ein erstes Ausführungsbeispiel eines Papillotoms mit einem gebeugten freien Endbereich, die beiden Schnittkanten sind zu erkennen,
- Fig. 2:: ein axiales Schnittbild durch den freien Endbereich des Katheters nach Fig. 1,
- Fig. 3:: ein axiales Schnittbild ähnlich Fig. 1 durch den freien Endbereich des zweiten Ausführungsbeispiels,
- Fig. 4:: einen radialen Schnitt entlang der Schnittlinie IV-IV in Fig. 3,
- Fig. 8:: eine perspektivische Darstellung einer PEG-Halteplatte und
- Fig. 9:: einen Schnitt durch eine Magenwand mit eingewachsener PEG-Halteplatte und PEG-Katheter, ein Papillotom ist eingeführt und schnittbereit.

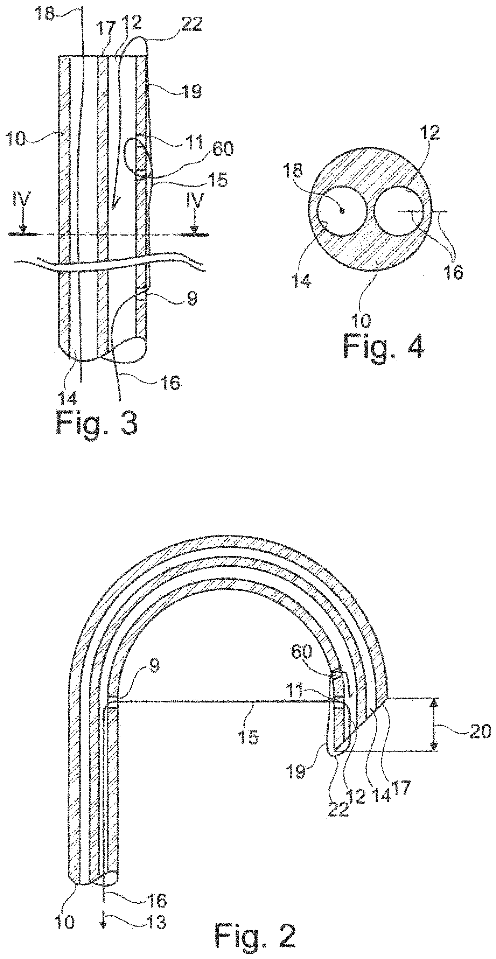

Das Papillotom hat einen Handgriff 2 mit einem stationären Teil 3 und einem bewegbaren Teil 4, das hier als Schieber ausgeführt ist, der am stationären Teil 3 verschiebbar geführt ist. Das stationäre Teil 3 hat eine Öse, am bewegbaren Teil 4 sind zwei entsprechende Öffnungen vorgesehen. In alle diese kann jeweils ein Finger bzw. ein Daumen gesteckt werden, durch Bewegen kann der bewegbare Teil 4 verschoben werden. Am bewegbaren Teil 4 ist ein HF-Anschluss 6 vorgesehen. Die Bewegung ist durch den Pfeil 7 dargestellt. Am unteren Ende des stationären Teils 3 geht dieses über in einen Katheter 10. Er ist zweilumig ausgeführt, siehe Figur 4. Ein zweites Lumen 14 ist über einen Ansatz 8 zugänglich. Dieser schließt mit einem Schraubgewinde ab. Hier kann ein Führungsdraht 18 eingeführt werden (Figur 3).

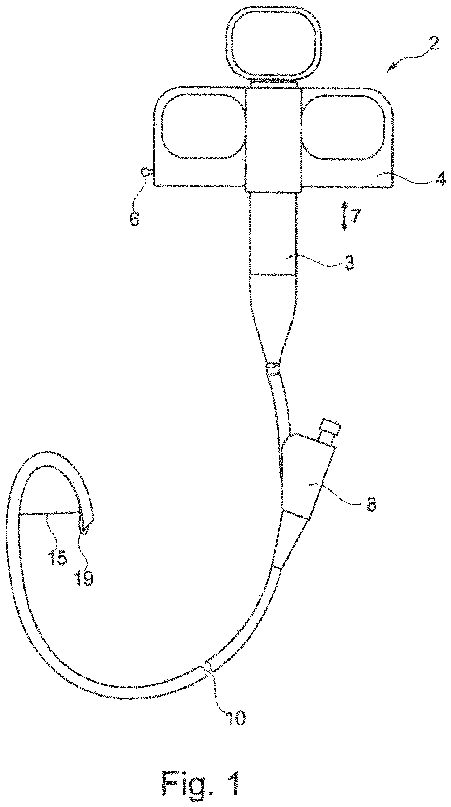

Der Katheter 10 hat einen freien Endbereich. Dieser erstreckt sich nach Figur 2 von einer vordersten Spitze des Papillotoms bis etwas über eine hintere Öffnung 9 hinaus. Der vordere Endbereich ist der Teil des Katheters 10, der aus einem gestreckten in einen gebeugten Zustand übergeführt werden kann, der gebeugte Zustand ist in den Figuren 1 und 2 gezeigt.

Aus Figur 2 sind Details des freien Endbereichs ersichtlich. In einem ersten Lumen 12 ist ein Schneidedraht 16 verschiebbar geführt. Er ist mit dem bewegbaren Teil 4 und dem HF-Anschluss 6 nach Figur 1 verbunden. In dem gebeugten Zustand, wie ihn Figur 2 zeigt, wirkt eine Zugkraft entsprechend des Pfeils13 am Schneidedraht 16. Der Schneidedraht 16 ist zwischen der hinteren Öffnung 9 und einer vorderen Öffnung 11 gespannt, er verläuft dort möglichst gradlinig, er bildet eine quer verlaufene Schnittkante 15 aus. Nach Passieren der vorderen Öffnung 11 verläuft der Schneidedraht 16 im ersten Lumen 12 zu einem freien Ende 17 hin, dort tritt er aus dem ersten Lumen 12 aus und ist um das dortige Schlauchende des Katheters 10 herumgebogen. Er ist an der Außenseite entlanggeführt. Er tritt durch eine dritte Öffnung 60 wieder in das erste Lumen 12 ein. Die dritte Öffnung 60 befindet sich zwischen der vorderen Öffnung 11 und der hinteren Öffnung 9. Sie ist in sehr kurzem Abstand von der vorderen Öffnung 11. Der Abstand liegt im Bereich von 1 bis 8 mm. Die dritte Öffnung 60 ist ebenso orientiert zum Katheter 10 wie die beiden anderen Öffnungen 9, 11. Nach Durchtritt durch die dritte Öffnung ist der Schneidedraht 16 tiefer in das erste Lumen 12 hineingesteckt und eventuell durch eine Abwinkelung (wie gezeichnet) gesichert.

Im zweiten Ausführungsbeispiel nach den Figuren 3 und 4 ist das freie Ende nicht in einem spitzen Winkel abgeschnitten, wie insbesondere aus Figur 2 ersichtlich ist, vielmehr ist es im Winkel von 90° abgeschnitten. Der Winkel gemäß Figur 2 kann zwischen 10 und 80° liegen, er liegt vorzugsweise bei 45°. In das zweite Lumen 14 ist ein Führungsdraht 18 eingebracht. Der Verlauf des Schneidedrahts 16 ist ähnlich wie im ersten Ausführungsbeispiel, jedoch mit folgender Abweichung: Nach Passieren der vorderen Öffnung 11 verläuft der Schneidedraht 16 im ersten Lumen 12 in Richtung der hinteren Öffnung 9 hin und tritt über die dritte Öffnung 60 aus dem ersten Lumen 12 aus, ist dann um das freie Ende 17 des Katheters 10 herumgebogen und in das erste Lumen 12 hineingesteckt. Darin hat er eine beliebige Länge. Er ist eventuell durch eine Abwinkelung gesichert. Diese kann mit dem kurzen Stück zwischen der vorderen Öffnung 11 und der dritten Öffnung 60 des Schneidedrahts 16 oder dem Stück des Schneidedrahts 16 jenseits der hinteren Öffnung 9 verhakt sein.

Mit 20 ist ein Überstand bezeichnet. Die Länge des Überstandes 20 ist im ersten Ausführungsbeispiel praktisch gleich der Länge zwischen einem Kreuzungspunkt der längs verlaufenden Schnittkante 19 mit der querverlaufenden Schnittkante 15 und einer Schneidspitze 22. Genau genommen ist der Überstand 20 geringfügig länger als der Abstand zwischen der vorderen Öffnung 11 und dem freien Ende, weil der Schneidedraht 16 um das Schlauchende des Katheters 10 herumgebogen ist und dort zur Länge des Überstands beiträgt. An seiner Umlenkung an der Spitze des freien Endes 17 bildet der Schneidedraht 16 die Schneidspitze 22.

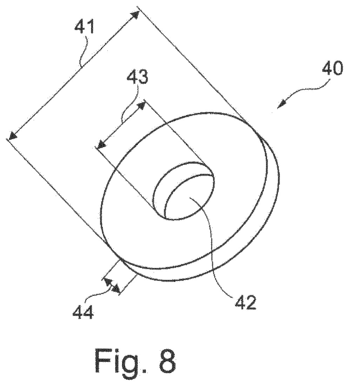

Die Figur 8 zeigt eine handelsübliche PEG-Halteplatte 40. Sie hat einen Durchgang 42. Sie ist als Kreisscheibe ausgebildet. Ihre Dicke wird mit 44 bezeichnet, 41 ist ihr Außendurchmesser, 43 der Lochdurchmesser.

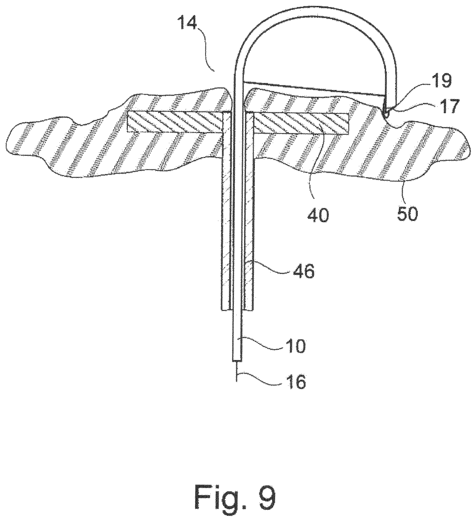

Figur 9 zeigt eine derartige PEG-Halteplatte 40, die in einer Magenwand 50 eingewachsen ist. Die PEG-Halteplatte 40 ist mit einem PEG-Katheter 46 verbunden. Erkennbar ist, dass der zuvor durch Mukosa überwucherte mageninnere Auslass des PEG-Katheters 46 geöffnet wurde, dies ist mittels der Schneidspitze 22 erfolgt. Das Papillotom ist bereits in einer Position gezeigt, in der ein Schneidvorgang erfolgen kann. Die Schneidspitze 22 ist in Kontakt mit der Muskosa, die quer verlaufende Schnittkante 15 liegt auf der Mukosa innenseitig auf. Das Mageninnere befindet sich in Figur 9 oben. Wird nun HF-Spannung an den Schneidedraht 16 gelegt und der Katheter 10 nach unten gezogen, kommt es zu einem Schneidevorgang. Dieser wird solange ausgeführt, bis die quer verlaufende Schnittkante 15 in Kontakt mit der PEG-Halteplatte 40 kommt. Dabei kann der Schneidedraht 16 noch weiter in Richtung des Pfeils 13 gezogen werden, um den Schneidvorgang durch die längs verlaufende Schnittkante 19 zu unterstützen. Dies kann fortgesetzt werden, bis es zu einem Kontakt mit dem Umfang der PEG-Halteplatte 40 kommt.

Beide Schnittkanten 15, 19 können somit separat und gezielt eingesetzt werden. Die quer verlaufende Schnittkante 15 wird gesteuert durch eine Bewegung des Katheters 10 in axialer Richtung und ein Drehen des Katheters 10 um die axiale Richtung. Die längs verlaufende Schnittkante 19 wird durch Anziehen in Richtung des Pfeils 13 und Loslassen des Schneidedrahtes 16 in Gegenrichtung hierzu gesteuert.

Ein Papillotom für die perkutane endoskopische Gastrostomie weist eine vordere Öffnung 11, die sich in einem freien Endbereich eines Katheters 10 und am Austritt eines ersten Lumens 12 befindet, eine hintere Öffnung 9, die weiter entfernt von einem freien Ende 17 ist als die vordere Öffnung 11 und die das erste Lumen 12 mit der Außenseite verbindet, und einen Schneidedraht 16 auf, der sich axial verschiebbar im ersten Lumen 12 befindet, der durch die vordere Öffnung 11 und die hinteren Öffnung 9 verläuft, der sich zwischen der vorderen Öffnung 11 und der hinteren Öffnung 9 auf der Außenseite befindet, wobei bei gespanntem Schneidedraht 16 der freie Endbereich bogenförmig verformt ist und der Schneidedraht 16 zwischen der vorderen Öffnung 11 und der hinteren Öffnung 9 eine quer verlaufende Schnittkante 15 bildet. Die vordere Öffnung 11 ist in einem Abstand von mindestens 3 mm vom freien Ende 17angeordnet ist. Zwischen dem freien Ende 17 und der vorderen Öffnung 11 ist ein Überstand 20 ausgebildet. Am freien Ende 17 des Katheters 10 ist eine Schneidspitze 20 ausgebildet, die mit dem Schneidedraht 16 verbunden ist und das vorderste Ende des Papillotoms bildet.

## Patentansprüche

1. Papillotom für die perkutane endoskopische Gastrostomie, das aufweist
- einen Handgriff (2)
- einen länglichen, biegbaren Katheter (10), der am Handgriff (2) befestigt ist, der einen freien Endbereich mit einem freien Ende (17) und der mindestens ein erstes Lumen (12) aufweist,
- eine vordere Öffnung (11), die sich im freien Endbereich befindet und das erste Lumen (12) mit einer Außenseite des Katheters (10) verbindet,
- eine hintere Öffnung (9), die sich im freien Endbereich des Katheters (10) befindet, die weiter entfernt vom freien Ende (17) ist als die vordere Öffnung (11) und die das erste Lumen (12) mit der Außenseite verbindet, wobei die vordere Öffnung (11) und die hintere Öffnung (9) in derselben Orientierung zum Katheter (10) angeordnet sind,
- einen Schneidedraht (16), der sich axial verschiebbar im ersten Lumen (12) befindet, der durch die vordere Öffnung (11) und die hinteren Öffnung (9) verläuft, der sich zwischen der vorderen Öffnung (11) und der hinteren Öffnung (9) auf der Außenseite des Katheters (10) befindet und der im freien Endbereich und im Handgriff (2) festgelegt ist, wobei bei betätigtem Handgriff (2) der Schneidedraht (16) gespannt ist, der freie Endbereich bogenförmig verformt ist und der Schneidedraht (16) zwischen der vorderen Öffnung (11) und der hinteren Öffnung (9) eine quer verlaufende Schnittkante (15) bildet,
wobei die vordere Öffnung (11) in einem Abstand von mindestens 3 mm vom freien Ende (17) angeordnet ist und zwischen dem freien Ende (17) und der vorderen Öffnung (11) ein Überstand (20) ausgebildet ist, **dadurch gekennzeichnet, dass** am freien Ende (17) des Katheters (10) eine Schneidspitze (20) ausgebildet ist, die mit dem Schneidedraht (16) verbunden ist und das vorderste Ende des Papillotoms bildet, und dass der Schneidedraht (16) sich zudem zwischen dem freien Ende (17) und der vorderen Öffnung (11) auf der Außenseite befindet, dort die Schneidspitze (20) und die längs verlaufende Schnittkante (19) bildet und dort in derselben Orientierung zum Katheter (10) wie die vordere Öffnung (11) und die hintere Öffnung (9) angeordnet ist.

2. Papillotom nach Anspruch 1, **dadurch gekennzeichnet, dass** der Schneidedraht (16) die Schneidspitze (20) ausbildet.

3. Papillotom nach einem der vorangegangenen Ansprüche, **dadurch gekennzeichnet, dass** das freie Ende (17) abgeschrägt ist, wobei eine Spitze der Abschrägung in derselben Orientierung zum Katheter (10) wie die vordere Öffnung (11) und die hintere Öffnung (9) angeordnet ist.

4. Papillotom nach einem der vorangegangenen Ansprüche, **dadurch gekennzeichnet, dass** die vordere Öffnung (11) in einem Abstand von maximal 15 mm vom freien Ende angeordnet ist, insbesondere dass sie in einem Abstand von 5 bis 10 mm und vorzugsweise von 4 bis 7 mm vom freien Ende (17) angeordnet ist.

5. Papillotom nach einem der vorangegangenen Ansprüche, **dadurch gekennzeichnet, dass** der Katheter (10) an einem stationären Teil (3) des Handgriffs (2) befestigt ist, und dass der Schneidedraht (16) an einem bewegbaren Teil (4) des Handgriffs (2) befestigt ist.

## Claims

1. Papillotome for percutaneous endoscopic gastrostomy, comprising
- a handle (2),
- an elongate flexible catheter (10) which is attached to the handle (2) and which has a free end region with a free end (17) and has at least one first lumen (12),
- a front opening (11) which is located in the free end region and connects the first lumen (12) to an outside of the catheter (10),
- a rear opening (9) which is located in the free end region of the catheter (10) and is further from the free end (17) than the front opening (11) and connects the first lumen (12) to the outside, wherein the front opening (11) and the rear opening (9) are disposed with the same orientation to the catheter (10),
- a cutting wire (16) which is located in an axially displaceable manner in the first lumen (12), extends through the front opening (11) and the rear opening (9) and is located between the front opening (11) and the rear opening (9) on the outside of the catheter (10), and which is fixed in the free end region and in the handle (2), wherein, when the handle (2) is actuated, the cutting wire (16) is tensioned, the free end region is deformed in an arc shape and the cutting wire (16) forms a transversely extending cutting edge (15) between the front opening (11) and the rear opening (9),
wherein the front opening (11) is disposed at a distance of at least 3 mm from the free end (17), and a projection (20) is formed between the free end (17) and the front opening (11), **characterized in that** a cutting tip (20) is formed at the free end (17) of the catheter (10), is connected to the cutting wire (16) and forms the foremost end of the papillotome, and **in that** the cutting wire (16) is moreover located on the outside between the free end (17) and the front opening (11), forms the cutting tip (20) there and the longitudinally extending cutting edge (19) and is disposed there with the same orientation to the catheter (10) as the front opening (11) and the rear opening (9).

2. Papillotome according to Claim 1, **characterized in that** the cutting wire (16) forms the cutting tip (20).

3. Papillotome according to one of the preceding claims, **characterized in that** the free end (17) is bevelled, wherein a tip of the bevel is disposed with the same orientation to the catheter (10) as the front opening (11) and the rear opening (9).

4. Papillotome according to one of the preceding claims, **characterized in that** the front opening (11) is disposed at a distance of at most 15 mm from the free end, in particular **in that** it is disposed at a distance of 5 to 10 mm and preferably of 4 to 7 mm from the free end (17).

5. Papillotome according to one of the preceding claims, **characterized in that** the catheter (10) is attached to a stationary part (3) of the handle (2), and **in that** the cutting wire (16) is attached to a movable part (4) of the handle (2).

## Revendications

1. Papillotome destiné à la gastrostomie endoscopique percutanée, ledit papillotome comprenant
- une poignée (2),
- un cathéter flexible allongé (10) qui est fixé à la poignée (2), qui comporte une région d'extrémité libre pourvue d'une extrémité libre (17) et qui comporte au moins une première lumière (12),
- une ouverture avant (11) qui est ménagée dans la région d'extrémité libre et qui relie la première lumière (12) à un côté extérieur du cathéter (10),
- une ouverture arrière (9) qui est ménagée dans la région d'extrémité libre du cathéter (10), qui est plus éloignée de l'extrémité libre (17) que l'ouverture avant (11) et qui relie la première lumière (12) au côté extérieur, l'ouverture avant (11) et l'ouverture arrière (9) étant disposées dans la même orientation par rapport au cathéter (10),
- un fil de coupe (16) qui est située de manière déplaçable axialement dans la première lumière (12), qui s'étend à travers l'ouverture avant (11) et l'ouverture arrière (9), qui est situé entre l'ouverture avant (11) et l'ouverture arrière (9) du côté extérieur du cathéter (10) et qui est fixée dans la région d'extrémité libre et dans la poignée (2), lorsque la poignée (2) est actionnée, le fil de coupe (16) étant tendu, la région d'extrémité libre étant déformée en forme d'arc et le fil de coupe (16) formant un bord de coupe transversal (15) entre l'ouverture avant (11) et l'ouverture arrière (9),
l'ouverture avant (11) étant disposée à une distance d'au moins 3 mm de l'extrémité libre (17) et une saillie (20) étant formée entre l'extrémité libre (17) et l'ouverture avant (11), **caractérisé en ce qu'**une pointe de coupe (20) est formée à l'extrémité libre (17) du cathéter (10), est reliée au fil de coupe (16) et forme l'extrémité la plus en avant du papillotome, et **en ce que** le fil de coupe (16) est également situé entre l'extrémité libre (17) et l'ouverture avant (11) du côté extérieur et forme à cet endroit la pointe de coupe (20) et le bord de coupe longitudinal (19) et est disposée à cet endroit dans la même orientation par rapport au cathéter (10) que l'ouverture avant (11) et l'ouverture arrière (9).

2. Papillotome selon la revendication 1, **caractérisé en ce que** le fil de coupe (16) forme la pointe de coupe (20).

3. Papillotome selon l'une des revendications précédentes, **caractérisé en ce que** l'extrémité libre (17) est biseautée, une pointe du biseau étant disposée dans la même orientation par rapport au cathéter (10) que l'ouverture avant (11) et l'ouverture arrière (9).

4. Papillotome selon l'une des revendications précédentes, **caractérisé en ce que** l'ouverture avant (11) est disposée à une distance maximale de 15 mm de l'extrémité libre, en particulier **en ce qu'**elle est disposée à une distance de 5 à 10 mm et de préférence 4 à 7 mm de l'extrémité libre (17).

5. Papillotome selon l'une des revendications précédentes, **caractérisé en ce que** le cathéter (10) est fixé sur une partie fixe (3) de la poignée (2), et **en ce que** le fil de coupe (16) est fixé sur une partie mobile (4) de la poignée (2).
